# EUROPEAN PATENT APPLICATION

(11) **EP 4 707 378 A2**
(43) Date of publication of application: **11.03.2026**
(21) Application number: 25221938.1
(22) Date of filing: 23.07.2019
(51) Int. Cl.: C12N 1/14

(54) **METHOD OF PRODUCING A MYCOLOGICAL PRODUCT AND PRODUCT MADE THEREBY**

(30) Priority: 23.07.2018 US 201862701906 P
(62) Divisional of application: 19841882.4
(71) Applicant: Ecovative LLC, Green Island, NY 12183 (US)
(72) Inventor: CARLTON, Alex, Troy, 12180 (US); BAYER, Eben, Troy, 12180 (US); MCINTYRE, Gavin, Troy, 12180 (US); KAPLAN-BIE, Jessie, Troy, 12180 (US)
(74) Representative: Mewburn Ellis LLP

(57) **Abstract**

A panel of mycological polymer consisting entirely of fungal mycelium as described in US Patent Application 16/190,585 is post-processed to impart desired characteristics thereto, such as, texture, flavor and nutritional profile for use as a foodstuff or a tissue scaffold. Alternatively, the growth conditions of the growth media may be tailored to obtain a desired density, morphology, and/or composition of the undifferentiated fungal material with or without the use of post-processes.

## Description

This application claims the benefit of US Provisional Patent Application 62/701,906, filed July 23, 2018.

This invention relates to a method of producing a mycological product and the product made thereby. More particularly, this invention relates to a method of producing mushroom mycelium as a nutritious matrix. Still more particularly, this invention relates to a method of producing mushroom mycelium as a nutritious matrix for cell cultures and for foodstuffs.

### Background of the invention

As is known from US Patent 9,485,917, a self-supporting composite material may be made of a substrate of discrete particles and a network of interconnected mycelia cells extending through and around the discrete particles and bonding the discrete particles together. In general, these composite materials may be classified as mycological biocomposites comprised of lignocellulosic waste materials, fungal cellular tissue, and potentially supplemental nutrients (minerals, vitamins, and the like).

As is known from published US Patent Application 2015/0033620, a mycological biopolymer product consisting entirely of fungal mycelium may be made by inoculating a nutritive substrate with a selected _{fun2gus} in a sealed environment except for a void space, which space is subsequently filled with a network of fungal mycelium. The environmental conditions for producing the mycological biopolymer product, i.e. a high carbon dioxide (CO₂) content i.e. from 5% to 7% by volume and an elevated temperature i.e. from 85° F. to 95° F., prevent full differentiation of the fungus into a mushroom. There are no stipe, cap, or spores produced. The biopolymer product grows into the void space of the tool, filling the space with an undifferentiated mycelium chitin-polymer, which is subsequently extracted from the substrate and dried.

As is also known from pending US Patent Application 16/190,585, filed November 14, 2018, another method of growing a biopolymer material employs incubation of a growth media comprised of nutritive substrate and a fungus in containers that are placed in a closed incubation chamber with air flows passed over each container while the chamber is maintained with a predetermined environment of humidity, temperature, carbon dioxide and oxygen. The mycological biopolymer is grown into a panel at a dry density of 0.5 to 4 pounds per cubic foot on a dry mass basis.

It is an object of the invention to provide a mycological biopolymer material for use in making functional products.

It is another object of the invention to provide a mycological biopolymer material that can be used to create a custom, mass-produced, non-animal matrix for the production of food, biomedical applications, and the like.

Briefly, the invention provides a method to create a custom, mass-produced, non-animal matrix for the production of food, biomedical applications, or the like.

In particular, the invention provides a method of producing a mycological product that comprises the step of growing a porous tissue of a mycological polymer consisting entirely of fungal mycelium on a growth media comprised of nutritive substrate and a fungus while preventing full differentiation of said fungus into a mushroom, such as described in US Patent Application 16/190,585, the disclosure of which is incorporated by reference herein. This step occurs within a closed incubation chamber maintained with a predetermined environment of humidity, temperature, carbon dioxide and oxygen sufficient to produce a mycelium biopolymer while preventing full differentiation of said fungus into a mushroom.

In accordance with the invention, the method includes the steps of removing a panel of mycological polymer from the porous tissue and packaging the panel for use.

The method (process) allows for the production of large, inert, tissue panels that can be further modified to generate a material with a custom texture, flavor, and nutritional profile for use as a foodstuff or a tissue scaffold.

The method involves tailoring the density, morphology, and composition of the undifferentiated fungal material during growth and/or the use of post-processes, to improve mouth-feel and/or affinity toward flavors, fats, cellular cultures, or the like.

In one embodiment, the growth conditions in the incubation chamber are altered to yield a well-aligned macromolecular structure, resembling meat, which can then be amended with flavorings and other additives including, but not limited to, proteins, fats, flavors, aromatics, heme molecules, micronutrients, and colorants.

In a second embodiment, flavorings and other additives are deposited on the growth media during the growth process, either through liquid or solid deposition, or though natural cellular uptake (bioadsorbtion), e.g., increasing mineral content in the growth media, to increase final content in the panel of tissue.

In a third embodiment, unwanted residues (e.g., malodors, enzymes that effect shelf-stability, and the like) are removed from the panel through either post-processing, or the altering of incubation conditions.

In a fourth embodiment, the incubation and/or post-process conditions are tuned to yield a panel of tissue that, texturally, resembles animal meat (e.g., increasing alignment and decreasing growth density via temperature and airflow controls and/or mechanically, enzymatically, or chemically altering the structure of the tissue.

In fifth embodiment, the panel of tissue (whole, or washed of any interfering residues) can be mechanically tenderized to density the native tissue (e.g., by mechanical compression, vacuum condensing, needling to entangle mycelium fibers) or to further orient fibers (e.g., calendar roller compression in the plane of fiber orientation). Additional ingredients such as a proteins, fats, flavors, aromatics, heme molecules, micronutrients, and colorants can be imparted into the mycelium matrix either before or immediately following the tenderization.

In a sixth embodiment, the panel of tissue (whole, or washed of any interfering residues) is used as a three-dimensional matrix in which non-fungal tissue cells can be supported and cultured, allowing for the in vitro production of tissue for meat consumption, or biomedical applications. This tissue can be engineered, using growth conditions or post-processing, to increase the affinity for desired cell growth (e.g., increasing or decreasing porosity, increasing or decreasing mycellial diameter, deacetylation of the chitin, and the like).

These and other objects and advantages will become more apparent from the following detailed description.

The method of producing a mycological product comprises an initial step of growing a porous tissue of a mycological polymer consisting entirely of fungal mycelium on a growth media comprised of nutritive substrate and a fungus while preventing full differentiation of said fungus into a mushroom, such as described in US Patent Application 16/190,585. This step occurs within a closed incubation chamber maintained with a predetermined environment of humidity, temperature, carbon dioxide and oxygen sufficient to produce a mycelium biopolymer while preventing full differentiation of said fungus into a mushroom. Thereafter, a panel of mycological polymer is removed from the porous tissue, for example, by slicing, and packaged for use.

The growth media may be as described in US Patent Application 16/190,585 or may be made basically of enzymatically available carbon and nitrogen sources (e.g., lignocellulosic biomass, chitinous biomass, carbohydrates) augmented with the additional micronutirents desired in the final product (e.g., minerals, vitamins).

Likewise, the fungal mycelium may be as described in US Patent Application 16/190,585 or may be made basically of an interconnected network of microscopic fibrils composed of chitin encapsulated in a matrix for beta glucans and protein.

In accordance with the method, the panel of mycological polymer is postprocessed to impart desired characteristics thereto. For example, the panel is infused with at least one additive selected form the group consisting of plant-derived proteins, fats, micronutrients and desired flavoring ingredients to mimic animal-derived meat products in said panel. Also, the additive may be a plant-derived additive, a cell derived additive, a fermented bacterial or fungal derived additive and an animal derived additive.

Alternatively, the growth conditions of the growth media may be tailored to obtain a desired density, morphology, and/or composition of the undifferentiated fungal material with or without the use of post-processes.

The following examples are provided to indicate the scope of the method. Example 1:
1. An 18-inch by 11-inch by 2.5-inch panel of the mycological biopolymer is grown under airflow (lateral flow less than or equal to 100 cubic feet per minute) and temperature conditions (greater than or equal to 85° Fahrenheit) designed to create a tender (i.e. easily macerated), porous tissue on a substrate composed of 15% crude protein, 33% non-fiber carbohydrates, 28% lignin and 14% crude fat.
2. The panel is extracted from the growth media via cutting and trimmed to desired size and shape.
3. The fresh panel is then vacuum infused with plant-derived proteins, fats, micronutrients and desired flavoring ingredients (e.g., bacon flavoring) to mimic animal-derived meat products.
4. The product is then vacuum packaged (with or without blanching) in sterile liquid and refrigerated until ready for consumption.

### Example 2:

1. An 18-inch by 11-inch by 2.5-inch panel of the mycological biopolymer is grown under airflow and temperature conditions, e.g. as above, designed to create a tender, porous tissue on a substrate composed of 15% crude protein, 33% non-fiber carbohydrates, 28% lignin, 14% crude fat, and elevated levels of essential dietary minerals.
2. During growth these elevated levels of minerals will be taken up by the fungus, bioaccumulating in the mycelial tissue, rendering the final product more nutritious and balanced.
3. The panel is extracted from the growth media via cutting and trimmed to desired size and shape.
4. The panel can then be further amended with desired additives via soaking or vacuum infusion
5. The panel can then be packaged, refrigerated, and consumed.

### Example 3:

1. An 18-inch by 11-inch by 2.5-inch panel of the mycological biopolymer is grown under airflow and temperature conditions, e.g. as above, designed to create a tender, porous tissue on a substrate composed of 15% crude protein, 33% non-fiber carbohydrates, 28% lignin and 14% crude fat.
2. During growth, desired nutrients, flavors, or other additives can be aerosolized into the growth chamber, condensing on the propagating tissue, and being incorporated into the matrix.
3. The panel is extracted from the growth media via cutting and trimmed to desired size and shape and packaged ready for consumption or cell culture.

### Example 4:

1. An 18-inch by 11-inch by 2.5-inch panel of the mycological biopolymer is grown under airflow and temperature conditions, e.g. as above, designed to create a tender, porous tissue on a substrate composed of 15% crude protein, 33% non-fiber carbohydrates, 28% lignin and 14% crude fat, with the addition of binding compounds, e.g. ligans and chelators, that target enzymes known to reduce shelf-life and resultant odors. These binding compounds act as blocking compounds that serve to increase shelf-life.
2. After panel extraction, the panel is washed in dilute hydrogen peroxide (3.5%) and dried under vacuum at 110C and 7 torr to remove known malodors (e.g., 2,4,6-Trichloroanisole)

### Example 5:

1. An 18-inch by 11-inch by 2.5-inch panel of the mycological biopolymer is grown under airflow and temperature conditions, e.g. as above, designed to create a tender, porous tissue on a substrate composed of 15% crude protein, 33% non-fiber carbohydrates, 28% lignin and 14% crude fat.
2. After panel extraction, the panel is mechanically tenderized with an array of pins and then subjected to a chitinase bath to further tenderize the tissue.
3. The panel is then packaged and is ready for consumption or cell culture.

### Example 6:

1. An 18-inch by 11-inch by 2.5-inch panel of the mycological biopolymer is grown under airflow (greater than or equal to 150 cubic feet per hour) and temperature conditions (less than or equal to 85° Fahrenheit) designed to create a dense (i.e. tough, gristle-like), porous tissue on a substrate composed of 15% crude protein, 33% non-fiber carbohydrates, 28% lignin and 14% crude fat.
2. The panel is decellularized in a heated SDS bath with sonication.
3. The decellularized panel is then sterilized, and inoculated with bovine myocytes in a bath of fetal bovine serum.
4. Cells are allowed to proliferate along the "scaffold" formed by the inoculated panel, until a complete three-dimensional cellular structure is formed in vitro. The "scaffold" in this instance is a matrix of interconnected mycelium fibrils between 1 and 10 microns in diameter and a porosity of no less than 75%. The fibers that compose the matrix serve as a structure for mammalian cells to adhere to, grow along, and differentiate from. The "scaffold" is the structure that mammalian and other cells are seeded onto and around to generate differentiated tissue structures.
5. This tissue is then useful in biomedical applications or for culinary purposes. For example, Other tissue engineering scaffolds include collagen and polylactic acid fibrils. Such scaffolds, as has been demonstrated with mycelium, can be seeded with osteoblasts (bone cells), allowed to grow on the mycelium under the right media and incubation conditions, and then differentiated into osteocytes that can be calcified to create bone tissue. The same is true for culinary approaches, but in this instance myocytes, or animal muscle cells (avian, bovine) are permitted to grow on and around the mycelium matrix. Incubated in grow media (fetal bovine serum was cited), and incubation conditions (typically the body temperature of the animal in question)

The method provides a panel of mycological biopolymer consisting entirely of fungal mycelium with an additive of at least one of plant-derived proteins, fats, micronutrients and selected flavoring ingredients therein.

In one embodiment, the additive mimics animal-derived meat products in the panel.

In another embodiment, the panel has inoculated bovine myocytes therein.

In still another embodiment, the panel contains compounds to block enzymes that would reduce shelf-life. (see Example 4).

The invention thus provides a mycological biopolymer material for use in making functional products. In particular, the invention provides a mycological biopolymer material that can be used to create a custom, mass-produced, non-animal matrix for the production of food, biomedical applications, and the like.

The following clauses further define the invention:
1. A method of producing a mycological product comprising the steps of
   growing a porous tissue of a mycological polymer consisting entirely of fungal mycelium on a growth media comprised of nutritive substrate and a fungus while preventing full differentiation of said fungus into a mushroom;
   removing a panel of mycological polymer from said porous tissue; and packaging said panel for use.
2. A method as set forth in clause 1 wherein said growth media is composed of 15% crude protein, 33% non-fiber carbohydrates, 28% lignin and 14% crude fat.
3. A method as set forth in clause 1 further comprising the step of infusing said panel with at least one additive selected form the group consisting of plant-derived proteins, fats, micronutrients and desired flavoring ingredients to mimic animal-derived meat products in said panel.
4. A method as set forth in clause 3 wherein said step of infusing is performed under vacuum.
5. A method as set forth in clause 3 wherein said step of infusing comprises soaking of said panel with said at least one additive.
6. A method as set forth in clause 3 wherein said at least one additive is one of a plant-derived additive, a cell derived additive, a fermented bacterial or fungal derived additive and an animal derived additive
7. A method as set forth in clause 1 further comprising the step of including elevated
   levels of essential dietary minerals in said growth media for bioaccumulating in said panel.
8. A method as set forth in clause 4 further comprising the step of adding flavoring additives to said panel by one of soaking and vacuum infusion.
9. A method as set forth in clause 1 further comprising the step of adding blocking compounds to said growth media to increase shelf-life in said panel.
10. A method as set forth in clause 6 further comprising the step of washing said panel in dilute hydrogen peroxide (3.5%) and drying under vacuum at a temperature and pressure to remove known malodors.
11. A method as set forth in clause 1 further comprising the steps of mechanically tenderizing said panel.
12. A method as set forth in clause 11 wherein said step of tenderizing includes passing an array of pins into said panel.
13. A method as set forth in clause 12 further comprising the step of thereafter placing said panel in a chitinase bath to further tenderize said panel.
14. A method as set forth in clause 1 further comprising the steps of:
   decellularizing said panel in a heated SDS bath with sonication;
   therefafter sterilizing said decellularized panel; and
   inoculating said sterilized panel with bovine myocytes in a bath of fetal bovine serum to form a complete three-dimensional cellular structure in vitro.
15. A panel of mycological biopolymer consisting entirely of fungal mycelium with an additive of at least one of plant-derived proteins, fats, micronutrients and selected flavoring ingredients therein.
16. A panel as set forth in clause 15 wherein said additive mimics animal-derived meat products in said panel.
17. A panel as set forth in clause 15 further comprising inoculated bovine myocytes therein.
18. A panel as set forth in clause 15 further comprising blocking compounds for increasing shelf-life in said panel

## Claims

1. A mycological biopolymer panel comprising a matrix of interconnected mycelium fibrils between 1 and 10 microns in diameter and a porosity of no less than 75%.

2. The mycological biopolymer panel of claim 1 wherein cells are proliferated on the mycological biopolymer.

3. The mycological biopolymer panel of claim 2 wherein the cells are osteoblasts, osteocytes or myocytes.

4. The mycological biopolymer panel of any one of claims 1 to 3 wherein the panel is infused with at least one additive selected from the group consisting of plant-derived proteins, fats, micronutrients and flavoring ingredients to mimic animal-derived meat products.

5. The mycological biopolymer panel of claim 4 wherein the additive is a plant-derived additive, a cell derived additive, a fermented bacterial or fungal derived additive or an animal derived additive.

6. The mycological biopolymer panel of claim 1 wherein the mycological biopolymer panel consists entirely of fungal mycelium with an additive of at least one of plant-derived proteins, fats, micronutrients and selected flavoring ingredients therein.

7. The mycological biopolymer panel of claim 6 wherein said additive mimics animal-derived meat products in said panel.

8. The mycological biopolymer panel of claim 2 further comprising inoculated bovine myocytes therein.

9. The mycological biopolymer panel of claim 1 further comprising blocking compounds for increasing shelf-life in said panel
